# DEMANDE DE BREVET EUROPEEN

(11) **EP 1 025 832 A1**
(43) Date de publication de la demande: **09.08.2000**
(21) Numéro de dépôt: 00400183.0
(22) Date de dépôt: 24.01.2000
(51) Int. Cl.: A61K 7/02, A61K 7/48

(54) **Composition cosmétique comprenant un poly (hydroxystyrène) dans un milieu aqueux**

(30) Priorité: 05.02.1999 FR 9901385
(71) Demandeur: L'OREAL, 75008 Paris (FR)
(72) Inventeur: Collin, Nathalie, 92330 Sceaux (FR); Yon, Maryline, 75013 Paris (FR)
(74) Mandataire: Kromer, Christophe

(57) **Abrégé**

L'invention a pour objet l'utilisation, dans une composition cosmétique ou pour la fabrication d'une composition topique, d'un poly(hydroxystyrène) pour obtenir un film résistant à l'eau et/ou au sébum et/ou aux frottements et/ou ne transférant pas et/ou ne migrant pas, la composition comprenant un milieu aqueux.

L'invention a aussi pour objet une composition à application topique, comprenant, dans un milieu aqueux physiologiquement acceptable, au moins un poly-(hydroxystyrène), un solvant dudit poly(hydroxystyrène), un polymère filmogène additionnel solubilisé ou dispersé dans le milieu aqueux, et une matière colorante.

Application au soin et au maquillage de la peau et des phanères.

## Description

La présente invention a pour objet l'utilisation dans une composition cosmétique, d'un poly(hydroxystyrène). L'invention a aussi pour objet une composition pour le soin ou le maquillage de la peau, y compris les lèvres, ou encore des phanères comme les cils, les sourcils, les cheveux et les ongles, notamment d'êtres humains, comprenant dans un milieu aqueux, un poly(hydroxystyrène), un solvant dudit poly(hydroxystyrène), un polymère filmogène additionnel, et une matière colorante.

Cette composition peut se présenter sous la forme de mascara, d'eye-liner, de produit pour les lèvres, de fard à joues ou à paupières, de fond de teint, de produit de maquillage du corps, de produit anti-cernes, de produit pour les ongles, de composition de protection solaire, de coloration de la peau, de produit de soin de la peau.

Les produits de maquillage ou de soin de la peau ou des lèvres d'êtres humains comme les eye-liners, les fonds de teints, les rouges à lèvres, ou bien encore des cils comme les mascaras, sont habituellement appliqués sous la forme d'une mince couche uniforme conduisant à la formation d'un film.

Pour favoriser la tenue du produit sur la peau et/ou les phanères, il est connu d'employer des polymères filmogènes. Pour des raisons d'innocuité, et en particulier pour éviter des problèmes d'allergies ou d'irritation dus à l'emploi de quantité importante de solvants organiques, la composition comprend généralement un milieu aqueux et le polymère filmogène utilisé est alors un polymère hydrosoluble ou bien encore un polymère sous forme de particules dispersées dans le milieu aqueux. Le film obtenu avec ces compositions aqueuses ne présente pas toujours une bonne résistance à l'eau, en particulier lorsque la composition comprend un polymère hydrosoluble, et le film au contact de l'eau, pendant la baignade ou la douche par exemple, se désagrège en partie en s'effritant ou bien encore en s'étalant. L'effritement du film engendre une perte sensible de l'intensité de la couleur du maquillage, obligeant ainsi la consommatrice à renouveler l'application du produit de maquillage. L'étalement du film forme, quant à lui, une auréole autour de la zone maquillée très inesthétique. Les larmes et la transpiration provoquent également ces mêmes inconvénients. Ceci est notamment les cas pour un eye-liner.

De plus, pour les peaux à tendance grasse, la sécrétion de sébum accentue le transfert du film de maquillage sur un support venant en contact avec la peau maquillée. Le film n'est donc pas non plus résistant au sébum. Par exemple, pour un eye-liner, lorsque le bord inférieur de la paupière supérieur vient en contact des autres parties de la paupière, le maquillage transfère sur cette partie de peau, provoquant des salissures inesthétiques et une diminution, voire une disparition, du maquillage, ce qui oblige la consommatrice à renouveler l'application du produit d'eye-liner. Ces désagréments sont encore plus accentués lors de frottements avec les doigts ou des tissus (serviettes, mouchoirs), provoquant une destruction importante, voire totale, du film déjà fragilisé.

En outre, ces compositions ont tendances à migrer dans les rides et ridules de la peau, en particulier autour des yeux et des lèvres, entraînant un effet inesthétique.

Le film de maquillage ne présente plus globalement une bonne tenue dans le temps.

La présente invention a donc pour but de proposer une composition à milieu aqueux ne présentant pas les inconvénients ci-dessus, et conduisant à la formation d'un film ayant une bonne tenue et résistant à l'eau et/ou aux frottements et/ou au sébum, ne transférant pas et ne migrant pas.

La demanderesse a constaté, de façon tout à fait surprenante, que l'utilisation d'un poly(hydroxystyrène), dans une composition physiologiquement acceptable comprenant un milieu aqueux pouvait permettre d'obtenir un film de très bonne tenue. Le film obtenu est notamment bien résistant à l'eau, lors de baignade ou de douche par exemple, et/ou aux larmes et/ou à la transpiration aux frottements par exemple des doigts ou des vêtements, et au sébum. Le film est également souple, flexible, non collant et possède des propriétés de non transfert et ne migre pas.

Les poly(para-hydroxystyrène) sont connus dans le document FR-A-2476441 comme avent antifongique et antibactérien. Le document EP-A-605951 décrit également leur utilisation dans une composition de vernis à ongles comprenant un poly(4-hydroxystyrène) pour améliorer l'adhésion sur l'ongle.

De façon plus précise, la présente invention a pour objet l'utilisation dans une composition cosmétique ou pour la fabrication d'une composition topique d'un poly(hydroxystyrène) éventuellement substitué, pour obtenir un film résistant à l'eau et/ou au sébum et/ou aux frottements et/ou ne transférant pas et/ou ne migrant pas, la composition comprenant un milieu aqueux.

Un autre objet de l'invention est une composition à application topique comprenant, dans un milieu aqueux physiologiquement acceptable, au moins un poly(hydrostyrène) éventuellement substitué, un solvant dudit poly(hydroxystyrène), un polymère filmogène additionnel solubilisé ou dispersé dans le milieu aqueux, et une matière colorante.

Par physiologiquement acceptable, il faut comprendre un milieu compatible avec la peau et/ou les fibres kératiniques, comme un milieu cosmétique.

L'invention a aussi pour objet un procédé cosmétique de maquillage de la peau et/ou des phanères consistant à appliquer sur ceux-ci une composition telle que décrite précédemment.

L'invention a également pour objet un procédé cosmétique pour limiter le transfert et/ou la migration et/ou pour améliorer la résistance aux frottements et/ou au sébum d'une composition, consistant à introduire dans ladite composition une quantité efficace d'un poly(hydroxystyrène) éventuellement substitué.

Le polymère filmogène utilisé dans la composition selon l'invention peut être un homopolymère ou un copolymère d'au moins un monomère hydroxystyrène éventuellement substitué. Dans la suite de la description, on utilisera le mot poly-(hydroxystyrène) pour tout polymère (homo- ou co-polymère) comportant au moins 2 motifs hydroxystyrène.

De préférence, le poly(hydroxystyrène) peut être un poly(para-hydroxystyrène) ou poly(4-hydroxystyrène), et notamment un homopolymère de para-hydroxystyrène (appelé également 4-hydroxystyrène). Dans cet homopolymère, les groupes phényle ne sont pas substitués.

Selon l'invention, au moins un des groupes phényle du poly(hydroxystyrène) peut être substitué par un ou plusieurs radicaux choisis dans le groupe formé par les radicaux alkyles en C₁-C₂₀, alcoxyalkyles en C₁-C₂₀, carboxyalkyles en C₁-C₂₀, phényle, phényle substitué, halogène (notamment chlore, brome, fluor et iode), benzotriazole, nitro, amino.

Par "phényle substitué", on entend un phényle substitué par au moins un substituant choisi dans le groupe formé par les radicaux halogènes (chlore, brome, fluor, iode), amino, nitro, hydroxy, alkyle en C₁-C₂₀, alcoxy (qui signifie un alcoxy linéaire ou ramifié ayant de 1 à 10 atomes de carbone), par exemple, méthoxy, éthoxy, propoxy, isopropoxy, butoxy, isobutoxy, sec-butoxy, tert-butoxy, pentyloxy, isopentyloxy, hexyloxy, heptyloxy, octyloxy, nonyloxy et décyloxy, alkyle halogéné (qui signifie un alkyle linéaire ou ramifié ayant de 1 à 8 atomes de carbone qui est substitué par au moins un halogène), par exemple, chlorométhyle, bromométhyle, fluorométhyle, iodométhyle, chloro-2 éthyle, bromo-2 éthyle, fluoro-2 éthyle, chloro-3 propyle, bromo-3 propyle, fluoro-3 propyle, chloro-4 butyle, fluoro-4 butyle, dichlorométhyle, dibromométhyle, difluorométhyle, diodométhyle, dichloro-2,2 éthyle, dibromo-2,2 éthyle, difluoro-2,2 éthyle, dichloro-3,3 propyle, difluoro-3,3 propyle, dichloro-4,4 butyle, difluoro-4,4 butyle, trichloro méthyle, difluoro-4,4 butyle, trichlorométhyle, trifluorométhyle, trifluoro-2,2,2 éthyle, trifluoro-2,3,3 propyle, tétrafluoro-1,1,2,2 éthyle, et tétrafluoro-2,2,3,3 propyle.

Par radical alkyle, on entend un radical alkyle linéaire ou ramifié en C₁-C₂₀, comme par exemple les radicaux méthyle, éthyle, propyle, isopropyle, butyle, isobutyle, sec-butyle, tertio-butyle, pentyle, isopentyle, néopentyle, hexyle, heptyle, octyle, éthyl-2 hexyle, 1,1,13,3-tétraméthylbutyle, nonyle, décyle, dodécyle, tétradécyle, nexadécyle, octadécyle et eicosyle.

Selon la présente invention, on entend aussi par poly(hydroxystyrène) des polymères, copolymères et polymères blocs ayant des motifs aromatiques comportant des groupements hydroxy dans leur structure. Ces polymères peuvent comporter d'autres motifs aromatiques ne comportant pas de groupement hydroxy, notamment lorsque le poly(hydroxystyrène) est issu de la copolymérisation de parahydroxystyrène et de styrène.

Les poly(hydroxystyrène) de la composition selon l'invention peuvent être linéaires ou branchés. On peut aussi utiliser un mélange de poly(hydroxystyrène) linéaire et de poly(hydroxystyrène) branché. Ces polymères sont connus et notamment décrits dans les documents US-A-5453483, US-A-5453481, US-A-5554719, US-A-5565544, EP-A-108624. De tels polymères sont vendus sous les dénominations "PHS-E", "PHS-8E01", "PHS-PG", "PHS-N", "PHS-PG-L" par la société CLARIANT.

Le PHS-E, PHS-8E01 et le PHS-PG-L sont un homopolymère linéaire répondant à la formule:

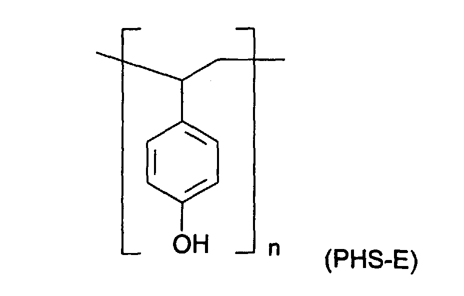

La masse moléculaire du PHS-E est comprise entre 8 000 et 100 000 g/mol.

Le PHS-PG est un polymère répondant à la formule:

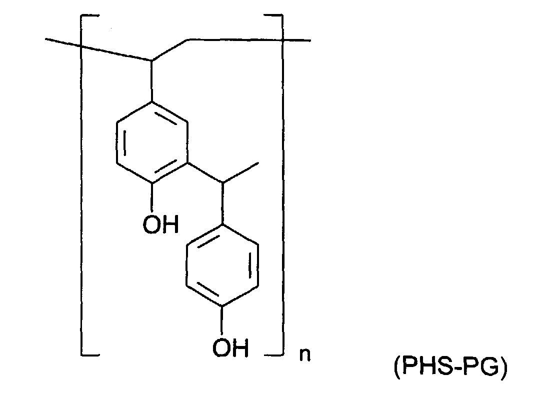

Sa masse moléculaire est comprise entre 4000 et 7 000 g/mol.

Le PHS-N est un polymère branché répondant à la formule:

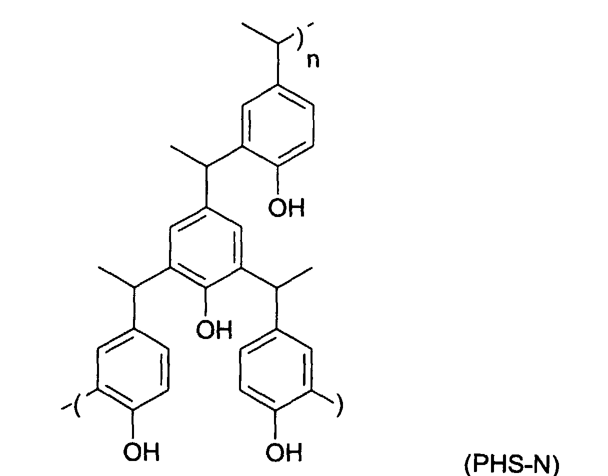

Sa masse moléculaire est comprise entre 4 000 et 7 000 g/mol.

Ledit poly(hydroxystyrène) peut être présent dans la composition en une teneur allant de 0,1 % à 30 % en poids, par rapport au poids total de la composition, de préférence de 1 % à 20 % en poids, et mieux de 1 % à 15 % en poids. Le poly(hydroxystyrène) peut être solubilisé ou dispersé sous forme de particules dans la composition selon les méthodes connues de l'homme du métier.

Le milieu aqueux (ou phase aqueuse) de la composition peut être constitué essentiellement d'eau. Il peut comprendre également un mélange d'eau et de solvant miscible à l'eau comme les monoalcools inférieurs ayant de 1 à 5 atomes de carbone, les glycols ayant de 2 à 8 atomes de carbone, les cétones en C₃-C₄, les aldéhydes en C₂-C₄.

Le poids total de la phase aqueuse dans la composition selon l'invention peut aller de 20 % à 99,9 % en poids, par rapport au poids total de la composition, de préférence de 40 % à 99,9 % en poids et mieux de 50 % à 95 % en poids.

La teneur en eau dans la composition peut aller de 10 % à 99,9 % en poids, par rapport au poids total de la composition, et de préférence de 20 % à 80 % en poids.

De préférence, la composition peut comprendre en outre au moins un solvant dudit poly(hydroxystyrène). Ce solvant peut être avantageusement un solvant polaire et de préférence miscible à l'eau. Le solvant peut être notamment choisi parmi les monoalcools en C₂-C₆, les glycols en C₂-C₈, les monoéthers de diol en C₃-C₆, les monoéthers de dialkylèneglycols en C₄-C₈, (le nombre de carbone indiqué correspondant au nombre total de carbone dans le composé). Comme solvant, on peut par exemple citer, l'éthanol, l'isopropanol, le propylène glycol, le butylène glycol, l'éthoxyéthanol, le diéthylène glycol. Le solvant peut être présent en une teneur allant de 0,1 % à 80 % en poids, par rapport au poids total de la composition, et de préférence de 1 à 60 % en poids.

Lorsque le solvant du poly(hydroxystyrène) n'est pas miscible à l'eau, on peut utiliser un cosolvant ou un émulsionnant.

La composition peut comprendre un polymère filmogène additionnel solubilisé ou dispersé dans le milieu aqueux de la composition, différent du poly(hydroxystyrène) défini précédemment.

Par "solubilisé dans le milieu aqueux", on entend un polymère qui peut être soluble dans l'eau ou dans le mélange d'eau et de solvant tel que défini précédemment.

Par "'dispersé dans le milieu aqueux", on entend un polymère insoluble dans l'eau ou le mélange d'eau et de solvants tels que définis précédemment, se présentant sous la forme de particules solides en dispersion dans le milieu aqueux. De telles dispersions peuvent être un latex, c'est-à-dire une dispersion obtenue par polymérisation en émulsion, ou bien encore un pseudolatex, c'est-à-dire une dispersion obtenue par mise en dispersion du polymère déjà synthétisé. Les techniques de préparation de ces dispersions sont bien connues de l'homme du métier.

Le polymère filmogène additionnel peut être choisi parmi:
- les dérivés de kératine, tels que les hydrolysats de kératine et les kératines sulfoniques ;
- les dérivés de chitine ou de chitosane anioniques, cationiques, amphotères ou non-ioniques ;
- les dérivés de cellulose tels que l'hydroxyéthylcellulose, l'hydroxypropylcellulose, la méthylcellulose, l'éthylhydroxyéthylcellulose, la carboxyméthylcellulose, ainsi que les dérivés quaternisés de la cellulose;
- les polymères ou copolymères acryliques, tels que les polyacrylates ou les polyméthacrylates ;
- les polymères vinyliques, comme les polyvinylpyrrolidones, les copolymères de l'éther méthylvinylique et de l'anhydride malique, le copolymère de l'acétate de vinyle et de l'acide crotonique, les copolymères de vinylpyrrolidone et d'acétate de vinyle; l'alcool polyvinylique ;
- les polyesters, notamment les polymères polyester et/ou polyesteramide anioniques dispersibles dans l'eau, comprenant des monomères portant une fonction : - SO₃M, avec M représentant un atome d'hydrogène, un ion ammonium NH₄⁺ ou un ion métallique, comme par exemple un ion Na⁺, Li⁺, K⁺, Mg²⁺, Ca²⁺, Cu²⁺, Fe²⁺, Fe³⁺. On peut citer en particulier les polymères décrits dans les documents US-3,734,874 ; US-4,233,196 ; US-4,304,901. Avantageusement, on choisit des polymères polyesters filmogènes à base d'au moins un acide dicarboxylique, d'au moins un diol et d'au moins un monomère aromatique bifonctionnel portant en outre un groupement -SO₃M tel que décrit ci-dessus ;
- les polyesters à chaîne grasse, les polyamides, et les résines époxyesters ;
- les polymères polyuréthannes, notamment les polyuréthannes anioniques, cationiques, non ioniques ou amphotères, les polyuréthannes-acryliques, les polyuréthannes-polyvinylpirrolidones, les polyester-polyuréthannes, les polyéther-polyuréthannes, les polyurées, les polyurée/polyuréthannes,
- les polymères d'origine naturelle, éventuellement modifiés, tels que :
   . les gommes arabiques, la gomme de guar, les dérivés du xanthane, la gomme de karaya ;
   . les alginates et les carraghénates ;
   . les glycoaminoglycanes, l'acide hyaluronique et ses dérivés ;
   . la résine shellac, la gomme de sandaraque, les dammars, les élémis, les copals ;
   . l'acide désoxyribonucléïque ;
   et leurs mélanges.

Le polymère filmogène additionnel peut être présent dans la composition en une teneur en matière sèche allant de 0,1 % à 10 % en poids, par rapport au poids total de la composition.

La composition peut également comprendre au moins une matière colorante comme les composés pulvérulents et/ou les colorants hydrosolubles et/ou les colorants liposolubles, par exemple à raison de 0,01 à 50 % du poids total de la composition. Les composés pulvérulents peuvent être choisis parmi les pigments et/ou les nacres et/ou les charges habituellement utilisés dans les compositions cosmétiques ou dermatologiques. Avantageusement, les composés pulvérulents représentent de 0,1 à 25 % du poids total de la composition et mieux de 1 à 20 %.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les charges peuvent être choisies parmi celles bien connues de l'homme du métier et couramment utilisées dans les compositions cosmétiques.

Parmi les colorants hydrosolubles, on peut citer le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle, leurs mélanges.

Parmi les colorants liposolubles, on peut citer le rouge Soudan, le DC Red 17, le DC Green 6, le β-carotène, l'huile de soja, le brun Soudan, le DC Yellow, 11, le DC Violet 2, le DC orange 5, le jaune quinoléine.

La composition peut comprendre, en outre, un ou plusieurs actifs notamment cosmétiques ou dermatologiques, tels que les agents hydratants, les vitamines, les acides gras essentiels, les protéines, les céramides, les filtres solaires, les agents anti-radicaux libres, les agents anti-inflamatoires. Bien entendu, l'homme du métier veillera à choisir ce ou ces éventuels composés complémentaires, et/ou leur quantité, de manière telles que les propriétés avantageuses de la composition pour l'utilisation selon l'invention ne soient pas, ou substantiellement pas, altérées par l'adjonction envisagée. Ces actifs peuvent être par exemple utilisés en une teneur allant de 0,001 % à 20 % en poids, par rapport au poids total de la composition.

La composition selon l'invention peut, de plus, comprendre selon le type d'application envisagée, les constituants classiquement utilisés dans les domaines considérés, qui sont présents en une quantité appropriée à la forme galénique souhaitée.

La composition peut contenir, en outre, tout additif usuellement utilisés dans de telles compositions tels que des épaississants, des parfums, des conservateurs, des tensioactifs, les huiles, les cires.

La composition peut se présenter sous forme de gel, de lotion, d'émulsion eau-dans-huile, huile-dans-eau, cire-dans-eau, eau-dans-cire, de dispersion vésiculaire contenant des lipides ioniques et/ou non ioniques.

Ces compositions à application topique peuvent constituer notamment une composition cosmétique, dermatologique, hygiénique ou pharmaceutique de protection, de soin ou de traitement de la peau, notamment pour le visage, pour le cou, pour les mains ou pour le corps (par exemple crème de soin, gel solaire, gel corporel), une composition de maquillage, de composition de protection solaire ou une composition de bronzage artificiel.

La composition de maquillage peut être notamment un mascara, un eye-liner, un produit pour les lèvres (rouge à lèvres), un fard à paupières ou à joues, un produit anti-cernes, un fond de teint, un produit pour les ongles (vernis-à-ongles ou base de soin), un produit de maquillage du corps du type tatouage provisoire ou semi-permanent.

L'invention est illustrée plus en détail dans les exemples suivants.

### Exemples 1 et 2 comparatifs:

On a préparé un eye-liner selon l'invention (exemple 1) et un eye-liner ne faisant pas partie de l'invention (exemple 2) ayant les compositions suivantes (les teneurs sont indiquées en gramme):

| **Ingrédients** | **Composition 1 (invention)** | **Composition 2 (hors invention)** |
|---|---|---|
| Alcool polyvinylique (Rhodoviol 4/125 de la société RHODIA CHIMIE) | 0,41 | 0,45 |
| Hydroxy propyl cellulose (Klucel H de la société HERCULES) | 0,015 | 0,017 |
| Copolymère vinylpyrrolidone/acétate de vinyle (70/30) dans l'éthanol à 50 % ( Luviskol VA73E de la société BASF) | 0,9 | 1 |
| Poly(para-hydroxystyrène) ("PHS-8E01 de la société CLARIANT) | 5 | - |
| Propylène glycol | 14,4 | 16 |
| Ethanol | 7,4 | 2,7 |
| Alcool oléylique oxyéthyléné (20 OE) | 4,1 | 4,5 |
| Silicate de magnésium et d'aluminium hydraté | 2 | 2,24 |
| Pigments noirs | 20,1 | 22,3 |
| Conservateurs | qs | qs |
| Eau | qsp 100 | qsp 100 |

La composition 1 contenant le poly(para-hydroxystyrène) s'applique facilement sur le bord des paupières et laisse, après application, un film homogène présentant des propriétés de tenue à l'eau et de résistance aux frottements des doigts et au sébum supérieures à celles du film obtenu avec la composition 2 ne contenant pas de poly(para-hydroxystyrène).

Les propriétés des compositions 1 et 2 ont également été évaluées in vitro. On a appliqué, pour chaque composition, une couche d'une épaisseur de 250 µm (avant séchage) sur 2 plaques de verres. Après séchage à température ambiante pendant 3 heures, on a conservé une plaque pendant 3 heures à nouveau à température ambiante et l'autre plaque à 45 °C à 80 % d'humidité relative.

On a évalué sur ces plaques la résistance aux frottements du doigt, à sec ou en présence d'eau ou de sébum.

En frottant le film déposé sur chaque plaque avec le doigt, on a constaté qu'avec la composition 1 de l'invention il n'y a aucune trace noire sur le doigt tandis qu'avec la composition 2 (hors invention), on a observé des traces noires sur le doigt.

En répétant la même opération de frottement en présence d'eau, on a constaté que pour la composition 1 de l'invention, le film ne se dissous pas et l'eau ne se colore pas, alors que pour la composition 2, le film se dissous et l'eau se colore en noir.

En effectuant également la même opération de frottement en ayant préalablement déposé du sébum reconstitué sur le film sec, on a constaté après frottements au doigt l'absence de traces noires sur le doigt avec la composition 1 selon l'invention tandis que la composition 2 laisse des traces noires sur le doigt.

Ainsi, les résultats obtenus montrent que la composition 1 selon l'invention permet d'obtenir un film ayant des propriétés de résistance aux frottements du doigt à sec ou en présence d'eau ou de sébum, supérieures aux propriétés du film obtenu avec la composition 2 (hors invention).

### Exemple 3:

On a préparé un eye-liner ayant la composition suivante :

| | |
|---|---|
| - Alcool polyvinylique (Rhodoviol 4/125 de RHODIA CHIMIE) | 0,4g |
| - Hydroxy propyl cellulose (Klucel H de la société HERCULES) | 0,015 g |
| - Copolymère vinylpyrrolidone/acétate de vinyle (70/30) dans l'éthanol à 50 % ( Luviskol VA73E de BASF) | 0,9 g |
| - Poly(para-hydroxystyrène) ("PHS-N de la société CLARIANT) | 2,5 g |
| - Copolymère acrylate d'éthyle/méthacrylate de méthyle (8/2) en dispersion aqueuse (Daitosol 5000 AD de DAITO) | 2,5 g MA |
| - Propylène glycol | 14,4 g |
| - Ethanol | 5,2g |
| - Alcool oléylique oxyéthyléné (20 OE) | 4 g |
| - Silicate de magnésium et d'aluminium hydraté | 2 g |
| - Pigments noirs | 20 g |
| - Conservateurs | qs |
| -Eau | qsp 100 g |

L'eye-liner s'applique facilement sur le bord des paupières et laisse après séchage un film homogène, résistant à l'eau, aux frottements des doigts et au sébum.

## Revendications

1. Utilisation, dans une composition cosmétique ou pour la fabrication d'une composition topique, d'un poly(hydroxy-styrène) éventuellement substitué pour obtenir un film résistant à l'eau et/ou au sébum et/ou aux frottements et/ou ne transférant pas et/ou ne migrant pas, la composition comprenant un milieu aqueux.

2. Utilisation selon la revendication 1, caractérisée par le fait que le poly(hydroxy styrène) est un poly(para-hydroxystyrène) éventuellement substitué.

3. Utilisation selon la revendication 1 ou 2, caractérisée par le fait qu'au moins un des groupes phényle du poly(hydroxystyrène) est substitué par un ou plusieurs radicaux choisis dans le groupe formé par les radicaux alkyles en C₁-C₂₀, alcoxyalkyles en C₁-C₂₀, carboxyalkyles en C₁-C₂₀, phényle, phényle substitué, halogène, benzotriazole, nitro, amino.

4. Utilisation selon l'une quelconque des revendications précédentes, caractérisée par le fait que le poly(hydroxystyrène) est un polymère linéaire ou branché.

5. Utilisation selon l'une quelconque des revendications précédentes, caractérisée par le fait que le poly(hydroxystyrène) est un mélange de polymère linéaire et de polymère branché.

6. Utilisation selon l'une quelconque des revendications précédentes, caractérisée par le fait que le poly(hydroxystyrène) a un poids moléculaire moyen en poids allant de 1000 à 500.000, de préférence de 1000 à 100.000, et mieux de 1000 à 30.000.

7. Utilisation selon l'une quelconque des revendications précédentes, caractérisée par le fait que le poly(hydroxystyrène) est présent en une teneur allant de 0,1 % à 30 % en poids, par rapport au poids total de la composition, de préférence de 1 % à 20 % en poids.

8. Utilisation selon l'une quelconque des revendications précédentes, caractérisée par le fait que la composition comprend de 20 % à 99,9 % en poids de phase aqueuse, par rapport au poids total de la composition, de préférence de 40 % à 99,9 % en poids, et mieux de 50 % à 95 % en poids.

9. Utilisation selon l'une quelconque des revendications précédentes, caractérisée par le fait que la composition comprend de l'eau en une teneur allant de 10 % à 99,9 % en poids, par rapport au poids total de la composition, et mieux de 20 % à 80 % en poids.

10. Utilisation selon l'une quelconque des revendications précédentes, caractérisée par le fait que la composition comprend au moins un solvant miscible à l'eau.

11. Utilisation selon l'une quelconque des revendications précédentes, caractérisée par le fait que la composition comprend au moins un solvant du poly-(hydroxystyrène).

12. Utilisation selon la revendication 11, caractérisée par le fait que le solvant est choisi dans le groupe formé par les monoalcools en C₂-C₆, les glycols en C₂-C₈, les monoéthers de diol en C₃-C₆, les monoéthers de dialkylèneglycols en C₄-C₈.

13. Utilisation selon la revendication 11 ou 12, caractérisée par le fait que le solvant est choisi dans le groupe formé par l'éthanol, l'isopropanol, le propylène glycol, le butylène glycol, l'éthoxyéthanol, le diéthylène glycol.

14. Utilisation selon l'une quelconque des revendications 11 à 13, caractérisée par le fait que le solvant du poly(hydroxystyrène) est présent en une teneur allant de 0,1 % à 80 % en poids, par rapport au poids total de la composition.

15. Utilisation selon l'une quelconque des revendications précédentes, caractérisée par le fait que la composition comprend en outre au moins un polymère filmogène additionnel solubilisé ou dispersé dans le milieu aqueux.

16. Utilisation selon l'une quelconque des revendications précédentes, caractérisée par le fait que la composition comprend au moins une matière colorante choisie dans le groupe formé par les composés pulvérulents, les colorants hydrosolubles et les colorants liposolubles.

17. Utilisation selon la revendication 16, caractérisée par le fait que la matière colorante comprend au moins un composé pulvérulent choisi dans le groupe formé par les charges, les pigments, les nacres et leurs mélanges.

18. Utilisation selon l'une quelconque des revendications précédentes, caractérisée par le fait que la composition comprend au moins un actif.

19. Utilisation selon l'une quelconque des revendications précédentes, caractérisée par le fait que la composition comprend au moins un additif choisi dans le groupe formé par les épaississants, les parfums, les conservateurs, les tensioactifs, les huiles, les cires.

20. Utilisation selon l'une quelconque des revendications précédentes, caractérisée par le fait que la composition se présente sous la forme de gel, de lotion, d'émulsion huile-dans-eau, d'émulsion eau-dans-huile, d'émulsion cire-dans-eau, d'émulsion eau-dans-cire, de dispersion vésiculaire contenant des lipides ioniques et/ou non ioniques.

21. Utilisation selon l'une quelconque des revendications précédentes, caractérisée par le fait que la composition se présente sous la forme d'un produit de soin et/ou de maquillage de la peau.

22. Utilisation selon l'une quelconque des revendications précédentes, caractérisée par le fait que la composition se présente sous la forme d'un mascara, d'un eye-liner, d'un produit pour les lèvres, d'un fard à paupières ou à joues, d'un produit anti-cernes, d'un fond de teint, d'un produit de maquillage du corps, de produit pour les ongles, de produit de protection, de soin ou de traitement de la peau, de composition de protection solaire, de composition de bronzage artificiel.

23. Composition à application topique, comprenant, dans un milieu aqueux physiologiquement acceptable, au moins un poly(hydroxystyrène) éventuellement substitué, un solvant dudit poly(hydroxystyrène), un polymère filmogène additionnel solubilisé ou dispersé dans le milieu aqueux, et une matière colorante.

24. Composition selon la revendication 23, caractérisée par le fait que le poly hydroxy styrène est un poly(para-hydroxystyrène) éventuellement substitué.

25. Composition selon la revendication 23 ou 24, caractérisée par le fait qu'au moins un des groupes phényle du poly(hydroxystyrène) est substitué par un ou plusieurs radicaux choisis dans le groupe formé par les radicaux alkyles en C₁-C₂₀, alcoxyalkyles en C₁-C₂₀, carboxyalkyles en C₁-C₂₀, phényle, phényle substitué, halogène, benzotriazole, nitro, amino.

26. Composition selon l'une quelconque des revendications 23 à 25, caractérisée par le fait que le poly(hydroxystyrène) est un polymère linéaire ou branché.

27. Composition selon l'une quelconque des revendications 23 à 26, caractérisée par le fait que le poly(hydroxystyrène) est un mélange de polymère linéaire et de polymère branché.

28. Composition selon l'une quelconque des revendications 23 à 27, caractérisée par le fait que le poly(hydroxystyrène) a un poids moléculaire moyen en poids allant de 1000 à 500.000, de préférence de 1000 à 100.000, et mieux de 1000 à 30.000.

29. Composition selon l'une quelconque des revendications 23 à 28, caractérisée par le fait que le poly(hydroxystyrène) est présent en une teneur allant de 0,1 % à 30 % en poids, par rapport au poids total de la composition, de préférence de 1 % à 20 % en poids.

30. Composition selon l'une quelconque des revendications 23 à 29, caractérisée par le fait qu'elle comprend de 20 % à 99,9 % en poids de phase aqueuse, par rapport au poids total de la composition, de préférence de 40 à 99,9 % en poids et mieux de 50 % à 95 % en poids.

31. Composition selon l'une quelconque des revendications 23 à 30, caractérisée par le fait qu'elle comprend de l'eau en une teneur allant de 10 % à 99,9 % en poids, par rapport au poids total de la composition, et mieux de 20 % à 80 % en poids.

32. Composition selon l'une quelconque des revendications 23 à 31, caractérisée par le fait que la composition comprend au moins un solvant miscible à l'eau.

33. Composition selon l'une quelconque des revendications 23 à 32, caractérisée par le fait que le solvant est choisi dans le groupe formé par les monoalcools en C₂-C₆, les glycols en C₂-C₈, les monoéthers de diol en C₃-C₆, les monoéthers de dialkylèneglycols en C₄-C₈.

34. Composition selon l'une quelconque des revendication 23 à 33, caractérisée par le fait que le solvant est choisi dans le groupe formé par l'éthanol, l'isopropanol, le propylène glycol, le butylène glycol, l'éthoxyéthanol, le diéthylène glycol.

35. Composition selon l'une quelconque des revendications 23 à 34, caractérisée par le fait que le solvant du poly(hydroxystyrène) est présent en une teneur allant de 0,1 % à 80 % en poids, par rapport au poids total de la composition.

36. Composition selon l'une quelconque des revendications 23 à 35, caractérisée par le fait que le polymère filmogène additionnel est choisi dans le groupe formé par les polymères dérivés de kératine, les polymères dérivés de chitine ou de chitosane, les polymères dérivés de cellulose, les polymères acryliques, les polymères vinyliques, les polyesters, les polyamides, les résines époxyesters, les polyuréthannes, les polyurées, les polymères d'origine naturelle, leurs mélanges.

37. Composition selon l'une quelconque des revendications 23 à 36, caractérisée par le fait que la matière colorante comprend au moins un composé pulvérulent, un colorant hydrosoluble ou un colorant liposoluble.

38. Composition selon l'une quelconque des revendications 23 à 37, caractérisée par le fait que la matière colorante comprend au moins un composé pulvérulent choisi dans le groupe formé par les charges, les pigments, les nacres et leurs mélanges.

39. Composition selon l'une quelconque des revendications 23 à 38, caractérisée par le fait qu'elle comprend au moins un actif.

40. Composition selon l'une quelconque des revendications 23 à 40, caractérisée par le fait qu'elle comprend au moins un additif choisi dans le groupe formé par les épaississants, les parfums, les conservateurs, les tensioactifs, les huiles, les cires.

41. Composition selon l'une quelconque des revendications 23 à 40, caractérisée par le fait qu'elle se présente sous la forme de gel, de lotion, d'émulsion huile-dans-eau, d'émulsion eau-dans-huile, d'émulsion cire-dans-eau, d'émulsion eau-dans-cire, de dispersion vésiculaire contenant des lipides ioniques et/ou non ioniques.

42. Composition selon l'une quelconque des revendications 23 à 41, caractérisée par le fait qu'elle se présente sous la forme d'un produit de soin et/ou de maquillage de la peau et/ou des phanères.

43. Composition selon l'une quelconque des revendications 23 à 42, caractérisée par le fait qu'elle se présente sous la forme d'un mascara, d'un eye-liner, d'un produit pour les lèvres, d'un fard à paupières ou à joues, d'un produit anti-cernes, d'un fond de teint, d'un produit de maquillage du corps, d'un produit pour les ongles, de produit de protection, de soin ou de traitement de la peau, de composition de protection solaire, de composition de bronzage artificiel.

44. Procédé cosmétique de maquillage de la peau et/ou des phanères, caractérisé par le fait que l'on applique sur la peau et/ou les phanères une composition selon l'une quelconque des revendications 23 à 43.

45. Procédé de traitement non thérapeutique de la peau et/ou des phanères, caractérisé par le fait que l'on applique sur la peau et/ou les phanères une composition selon l'une quelconque des revendications 23 à 43.

46. Procédé cosmétique pour limiter le transfert et/ou la migration et/ou pour améliorer la résistance aux frottements et/ou au sébum d'une composition, consistant à introduire dans ladite composition une quantité efficace d'un poly-(hydroxystyrène) éventuellement substitué.
